(19) Europäisches Patentamt
European Patent Office
Office européen des brevets

(11) Numéro de publication : **0 005 098**
**B1**

(12) # FASCICULE DE BREVET EUROPÉEN

(45) Date de publication du fascicule du brevet : **03.02.82**

(51) Int. Cl.³ : **C 07 C 179/22**, C 11 D 3/39

(21) Numéro de dépôt : **79400219.6**

(22) Date de dépôt : **04.04.79**

(54) **Compositions contenant un mono- ou polyalkylarylsulfonate de métal alcalin et l'hydroperoxyde correspondant, procédé pour leur préparation et compositions détergentes les contenant.**

(30) Priorité : **14.04.78 FR 7811082**
**10.10.78 FR 7828907**

(43) Date de publication de la demande : **31.10.79 (Bulletin 79/22)**

(45) Mention de la délivrance du brevet : **03.02.82 Bulletin 82/05**

(84) Etats contractants désignés : **BE DE GB IT LU NL**

(56) Documents cités :
**FR - A - 2 088 716**
**GB - A - 889 652**
**US - A - 2 829 158**

(73) Titulaire : **Société Chimique des Charbonnages**
**Tour Aurore Place des Reflets Cedex no 5**
**F-92080 Paris La Défense 2 (FR)**

(72) Inventeur : **Couderc, Pierre**
**78, rue Emile Zola**
**F-62400 Bethune (FR)**
Inventeur : **Grardel, Jean-Luc**
**27, rue du Capitaine**
**Coussette-Annezin F-62400 Bethune (FR)**
Inventeur : **Caux, Gérard**
**45, rue des Marronniers**
**Aix Noulette F-62160 Bully Les Mines (FR)**

**EP 0 005 098 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Compositions contenant un mono- ou polyalkylarylsulfonate de métal alcalin et l'hydroperoxyde correspondant, procédé pour leur préparation et compositions détergentes les contenant

La présente invention concerne des compositions solides comprenant un mono- ou polyalkylarylsulfonate de métal alcalin et l'hydroperoxyde correspondant ; elle concerne également un procédé perfectionné pour la préparation des mélanges de mono- ou polyalkyarylsulfonate de métal alcalin et de l'hydroperoxyde correspondant et un procédé pour la préparation desdits mélanges à l'état sec ; elle concerne enfin l'utilisation de l'hydroperoxyde comme agent de blanchiment et les compositions détergentes solubles dans l'eau contenant les compositions solides comprenant un mono- ou polyalkylarylsulfonate de métal alcalin et l'hydroperoxyde correspondant.

La synthèse de composés arylsulfonate alcalin comportant, sur le noyau aryl, une fonction hydroperoxyde ROOH a été relativement peu étudiée ; cependant le brevet des Etats-Unis d'Amérique n° 2.829.158 décrit un procédé pour la préparation de composés de ce type et de mélanges en solution aqueuse, d'alkylarylsulfonate peroxyde et d'alkylarylsulfonate. Ce même brevet précise, sans donner d'exemple spécifique, qu'il serait éventuellement possible de préparer, à partir de solutions aqueuses ainsi préparées, l'hydroperoxyde à l'état pur à condition d'évaporer le solvant dudit hydroperoxyde à une température très basse par exemple inférieure à −10 °C.

Or, il peut être intéressant de disposer d'un procédé industriel permettant la préparation, à l'état sec, des mélanges de mono- ou polyalkylarylsulfonates de métal alcalin et d'hydroperoxydes correspondants, lesdits mélanges secs étant notamment utilisables dans des compositions détergentes, comme il sera décrit ci-dessous, ou comme initiateurs de réactions radicalaires.

La présente invention a pour premier objet une composition comprenant un mono- ou polyalkylarylsulfonate de métal alcalin de formule :

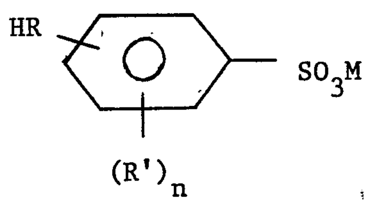

dans laquelle M est un métal alcalin, RH est un radical alkyle inférieur, R' est un radical alkyle inférieur, n est un nombre entier inférieur à 5, et un hydroperoxyde de formule :

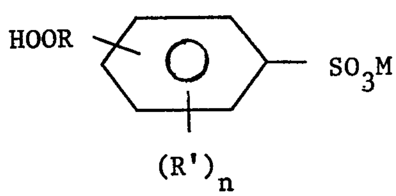

dans laquelle M, RH, R' et n ont les mêmes significations que ci-dessus, caractérisée en ce qu'elle se présente à l'état solide et sec et en ce qu'elle contient :

— de 5 à 75 % en poids dudit mono- ou polyalkylarylsulfonate de métal alcalin,

— de 25 à 70 % en poids dudit hydroperoxyde,

— de 0 à 30 % en poids de l'alcool provenant de la décomposition dudit hydroperoxyde et ayant la formule :

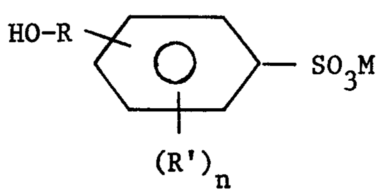

dans laquelle M, RH, R' et n ont les mêmes significations que ci-dessus.

La structure de l'hydroperoxyde contenu dans une composition selon l'invention est mise en évidence par RMN du Carbone 13. Dans le cas où RH est le radical isopropyle, la fréquence de résonance du carbone central dudit radical isopropyle $—CH(CH_3)_2$ qui est d'environ 34 ppm pour l'hydrocarbure passe à 83 ppm pour l'hydroperoxyde et à 72 ppm pour l'alcool provenant de la composition de l'hydroperoxyde.

La présente invention a pour second objet un procédé de peroxydation d'un mono- ou polyalkylarylsulfonate de métal alcalin de formule :

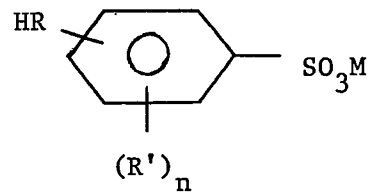

dans laquelle M est un métal alcalin, RH est un radical alkyle inférieur, R' est un radical alkyle inférieur, n est un nombre entier inférieur à 5, par réaction, en solution aqueuse, avec de l'oxygène, caractérisé en ce que :

— la réaction est réalisée à une température non supérieure à 85 °C,

— la réaction est effectuée à pH compris entre 9 et 10,5,

— on utilise comme catalyseur de 1 à 50 ppm de cobalt, par rapport au mono- ou polyalkylarylsulfonate de métal alcalin.

De préférence M sera le sodium, RH le radical isopropyle, R' sera choisi parmi les radicaux méthyle, éthyle, n-propyle et isopropyle, n sera égal à 0 ou 1 et le cobalt sera sous forme de naphténate de cobalt.

La préparation des hydroperoxydes des mono- ou polyalkylarylsulfonates de métal alcalin en milieu aqueux par mise en contact avec de l'oxygène (soit de l'oxygène pur, soit de l'air, soit de l'air enrichi en oxygène) est grandement facilitée si l'on utilise comme catalyseur du naphténate de cobalt. On a remarqué qu'en utilisant ce catalyseur on pouvait opérer le peroxydation à une température de 80 à 85 °C au lieu de 90-95 °C. Et ceci est d'autant plus souhaitable que, à des températures supérieures à 85 °C, la décomposition de l'hydroperoxyde même en milieu alcalin n'est pas négigeable. La présence du naphténate de cobalt permet donc de mieux utiliser l'oxygène nécessaire à cette réaction et d'éviter la formation trop importante de l'alcool provenant de la décomposition de l'hydroperoxyde. Cependant, il faut éviter la présence de quantités trop grandes de naphténate de cobalt. La demanderesse a en effet trouvé que pour des quantités supérieures à 50 ppm (exprimés en cobalt) par rapport au sulfonate de départ, le naphténate catalysait la décomposition de l'hydroperoxyde. Ainsi la teneur optimale en naphténate de cobalt se situe entre 1 et 50 ppm de cobalt en poids par rapport au sulfonate de départ.

La réaction de peroxydation a lieu en milieu légèrement alcalin et de préférence à un pH compris entre 9 et 10,5. En effet en deçà et au-delà de ces valeurs la décomposition de l'hydroperoxyde en alcool n'est pas négligeable.

La présence de peroxydes et notamment d'eau oxygénée favorise l'initiation de la peroxydation et une quantité de l'ordre de 10 à 20 parties en poids d'eau oxygénée à 50 % en poids, pour 100 parties en poids de sulfonate de départ, s'avère intéressante.

En effectuant la peroxydation à 80 °C, à pH 10, en présence de moins de 10 ppm de cobalt, sous forme de naphténate de cobalt, par rapport au mono- ou polyalkylarylsulfonate de départ, on obtient, en limitant volontairement le taux de conversion du sulfonate à une valeur assez faible, un milieu réactionnel qui, après séchage sous vide à 20 °C, fournit une poudre contenant environ 85 à 90 % de sulfonate non transformé, 10 à 15 % de l'hydroperoxyde correspondant et dans laquelle la présence d'alcool de décomposition de l'hydroperoxyde n'a pu être détectée. On peut obtenir une poudre ayant une teneur plus élevée en hydroperoxyde en soumettant le mélange réactionnel à une cristallisation fractionnée telle que décrite ci-après, et en répétant au besoin cette opération sur le filtrat obtenu.

La titulaire a trouvé que pour obtenir des produits industriellement intéressants il était souhaitable de poursuivre la réaction de peroxydation jusqu'à ce que l'extrait sec de la solution aqueuse obtenue contienne au moins 25 % environ, en poids, de l'hydroperoxyde. Par contre ladite peroxydation ne doit pas être poursuivie au-delà d'un taux global de transformation du sulfonate voisin de 50 %, afin d'éviter une décomposition trop importante de l'hydroperoxyde formé en alcool, qui diminue la sélectivité de la réaction de peroxydation du sulfonate de départ, donc le rendement en hydroperoxyde par rapport à l'oxygène absorbé. Ce rendement est supérieur ou égal à 75 % pour des taux globaux de transformation du sulfonate de départ n'excédant pas 50 %.

Les sulfonates de sodium utilisés comme matière première sont préparés de façon connue en soi par traitement de l'hydrocarbure correspondant par l'acide sulfurique concentré à chaud, neutralisation par la soude, refroidissement, filtration et séchage du précipité obtenu.

Les peroxydations des mono- ou polyalkylarylsulfonates de sodium ainsi obtenus sont conduites avec un réacteur tubulaire chauffé, fonctionnant en circuit fermé avec recyclage de la phase gazeuse dans laquelle on injecte un courant gazeux d'oxygène. L'absorption de l'oxygène en fonction du temps se fait suivant une courbe en S avec des périodes d'induction relativement courtes. Le débit de la phase gazeuse, qui circule en recyclage continu à travers le masse réactionnelle, est d'environ 80 à 90 litres par heure.

La teneur en hydroperoxyde du mélange réactionnel et des produits secs est mesurée par voie chimique (iodométrie).

La teneur en alcool provenant de la décomposition de l'hydroperoxyde est mesurée par RMN du

proton ($CH_3$ adjacents du carbone portant la fonction OH).

Une composition sèche selon l'invention est pratiquement stable à température ambiante et peut en principe être obtenue par une simple évaporation, à sec, de la solution obtenue en mettant en œuvre le procédé décrit ci-dessus mais, étant donné que dans la réaction de peroxydation on ne peut atteindre des taux de conversion en peroxyde, très élevés, il sera en fait difficile d'obtenir des produits secs ayant un taux de peroxyde assez élevé.

Il a été trouvé que la préparation industrielle d'une composition sèche définie ci-dessus pouvant avantageusement être réalisée en traitant le mélange réactionnel obtenu en fin de réaction de peroxydation de façon à provoquer une cristallisation partielle de ce mélange, en filtrant, la suspension ainsi obtenue et en évaporant à sec le filtrat à une température maximale de 40 °C, sous vide. Il a été trouvé en effet que l'alkylarylsulfonate utilisé comme produit de départ était moins soluble dans l'eau ou dans les alcools inférieurs, que l'hydroperoxyde correspondant. Cette cristallisation fractionnée peut être répétée si on le désire sur le filtrat obtenu après, par exemple, évaporation partielle de l'eau.

Pour réaliser cette cristallisation partielle on pourra par exemple utiliser un des moyens suivants : refroidissement du milieu réactionnel entre 0 et 20 °C ou concentration à 40 °C sous vide et refroidissement entre 0 et 20 °C. On pourra également concentrer à sec le mélange réactionnel (et éventuellement utiliser directement le mélange sec obtenu, pour les applications prévues), le reprendre par du méthanol, refroidir la suspension aux environs de 5 °C et filtrer le précipité. Le filtrat, enrichi en hydroperoxyde est alors concentré à sec à une température maximale de 40 °C, sous vide.

Dans leur application comme constituants de compositions détergentes, les mélanges solides et secs de mono- ou polyalkylarylsulfonate de métal alcalin et de l'hydroperoxyde correspondant permettent pour l'essentiel un remplacement au moins partiel du perborate de sodium ou des produits équivalents qui, dans les compositions détergentes connues, jouent le rôle d'agent de blanchiment.

Les détergents pulvérulents utilisés habituellement en solution aqueuse pour le lavage des fibres textiles à haute température contiennent généralement comme agent de blanchiment des persels minéraux. Parmi ceux-ci, un des plus couramment utilisés est le perborate de sodium, qui peut représenter jusqu'à 30 % en poids de la poudre de lavage.

On peut montrer que dans cette application l'hydroperoxyde contenu dans les mélanges solides et secs selon l'invention joue le rôle principal d'agent de blanchiment ; les autres composants de ces mélanges jouent également très probablement des rôles bénéfiques notamment dans les propriétés secondaires (assouplissantes, tensio-actives) des détergents obtenus.

Compte tenu de la stabilité des produits contenus dans ces mélanges, notamment de l'hydroperoxyde, les détergents qui seront obtenus seront plus particulièrement destinés, pour ce qui concerne leur activité découlant de la présence dudit hydroperoxyde, aux lavages aux températures élevées (par exemple vers 90 °C).

La présente invention a pour troisième objet des compositions détergentes caractérisées en ce qu'elles contiennent, en tant qu'agent de blanchiment, un hydroperoxyde d'un mono- ou polyalkylarylsulfonate de métal alcalin de formule :

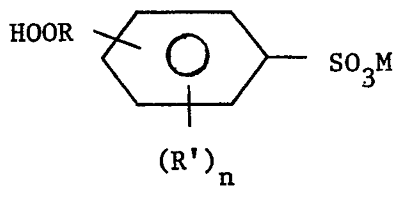

dans laquelle M est un métal alcalin, RH est un radical alkyle inférieur, R' est un radical alkyle inférieur, n est un nombre entier inférieur à 5.

L'hydroperoxyde préféré est l'hydroperoxyde de l'isopropylbenzène sulfonate de sodium (hydroperoxyde de cumène sulfonate de sodium).

Parmi les produits contenant l'hydroperoxyde, on utilisera avantageusement les mélanges stables contenant de 25 à 70 % en poids d'hydroperoxyde d'alkylarylsulfonate de métal alcalin, de 5 à 75 % en poids d'un alkylarylsulfonate de métal alcalin, et jusqu'à 30 % en poids d'alcool provenant de la décomposition dudit hydroperoxyde. L'alkylarylsulfonate de métal alcalin est très généralement constitué par le produit ayant été utilisé comme produit de départ pour la préparation dudit hydroperoxyde.

Ces mélanges pourront remplacer poids pour poids, dans les compositions détergentes, le perborate de sodium. C'est ainsi qu'on pourra préparer un détergent contenant jusqu'à 30 % en poids desdits mélanges ; cependant, compte tenu de l'efficacité desdits mélanges comme détergents et agents de blanchiment, il est généralement souhaitable de n'utiliser dans les détergents que des quantités desdits mélanges qui correspondent à une présence dans la poudre détergente, de 0,1 à 5 % en poids de l'hydroperoxyde.

Il est également possible que les mélanges utilisés selon l'invention ne remplacent que partiellement le perborate habituellement utilisé ; notamment il est apparu souhaitable d'employer simultanément d'une part, les mélanges selon l'invention de façon à apporter environ 2 à 4 % en poids d'hydroperoxyde et

d'autre part, du perborate de sodium à raison de moins de 5 % en poids et de préférence environ 2 % par rapport à la composition détergente.

Avec d'aussi faibles proportions d'agent de blanchiment, la disparition des salissures du linge est complète.

L'efficacité des poudres de lavage est déterminée par l'état de blancheur obtenu par les tissus lavés. Ce degré de blancheur est du même ordre de grandeur, à taux d'oxygène actif égal, pour les compositions détergentes selon l'invention, et qui, de plus, contiennent peu d'hydroperoxyde, et pour les lessives à faible taux de perborate. Il est sensiblement équivalent pour les compositions détergentes à faible teneur en hydroperoxyde, par rapport aux poudres détergentes classiques contenant environ 30 % de perborate de sodium. Il est possible d'améliorer les résultats obtenus avec les compositions détergentes contenant peu d'hydroperoxydes à condition de leur incorporer des traces de sels cuivriques (par exemple le sulfate de cuivre) en quantités telles que la teneur exprimée en cuivre soit inférieure à 1 000 ppm, et de préférence comprise entre 100 et 800 ppm par rapport à la poudre détergente. Ce fait est assez surprenant dans la mesure où l'addition de traces de sels cuivriques à une poudre détergente à base de perborate diminue l'efficacité de cette poudre. En particulier, il est donc possible avec les compositions détergentes selon l'invention d'utiliser sans inconvénient les eaux d'alimentation contenant des traces de sels de cuivre (soit par nature, soit par suite de l'utilisation de canalisation en cuivre). Cependant, la demanderesse a remarqué que cette présence de sels cuivriques est bénéfique pour certaines salissures (en particulier la teinture au noir Immédial) mais est défavorable si le textile à laver a été taché de vin. Dans ce dernier cas, il est possible d'améliorer le degré de blancheur du textile lavé en ajoutant préalablement, à la poudre détergente, un complexant du cuivre. Parmi les complexants connus du cuivre plus particulièrement utilisables dans la présente invention on peut citer l'acide nitrilotriacétique (NTA) et/ou un de ses sels alcalins et l'acide éthylènediaminetétracétique (EDTA) et/ou un de ses sels alcalins ou un mélange de ces divers complexants ; compte tenu des quantités de cuivre utilisables on emploiera généralement de 0,1 à 5 % (et de préférence 0,5 à 2 % en poids), par rapport à la poudre détergente, de NTA et de 0,1 à 2 % en poids, par rapport à la poudre détergente, de EDTA.

Les compositions détergentes en poudre selon la présente invention contiendront donc :

— d'une part, les mélanges solides et secs contenant un mono- ou polyalkylarylsulfonate de métal alcalin, l'hydroperoxyde correspondant et éventuellement l'alcool provenant de la décomposition de l'hydroperoxyde,

— d'autre part, si nécessaire, les additifs cuivre et complexants mentionnés ci-dessus et,

— également les ingrédients connus utilisés dans les détergents tels que un ou plusieurs détergents anioniques comme le dodécylbenzènesulfonate de sodium, un ou plusieurs savons comme le sel de sodium de suif, un détergent non ionique (dérivé polyoxyéthyléné ou polyoxypropyléné), du tripolyphosphate de sodium agissant comme séquestrant, et du silicate de sodium.

De plus lesdites compositions détergentes solides pourront également contenir :

— de faibles quantités d'une base (par exemple de la soude) de façon à ce que, lors de l'utilisation desdites poudres, les solutions obtenues aient un pH convenable (de préférence de 9 à 12),

— du sulfate de sodium et/ou du carbonate de sodium, produits utilisés comme charges et,

— des produits tels que des azurants optiques, des parfums...

Il a été constaté d'ailleurs que l'utilisation des mélanges solides et secs selon l'invention dans les compositions détergentes, permet de diminuer ou même de supprimer l'emploi, dans lesdites compositions détergentes, du dodécylbenzène sulfonate de sodium (ou du produit équivalent) généralement présent dans la plupart des compositions détergentes. Cette diminution (ou suppression) du dodécylbenzène sulfonate de sodium est possible notamment dans les compositions détergentes où tout ou partie du perborate a été remplacé par les mélanges contenant l'hydroperoxyde, le sulfonate et éventuellement l'alcool.

Les compositions détergentes selon l'invention procurent en outre un résultat inattendu : la demanderesse a constaté qu'elles possèdent un certain effet assouplissant permettant d'éviter ainsi au rinçage l'addition de produits connus pour redonner au linge lavé sa souplesse ; cette action assouplissante semble provenir de l'alcool présent dans les « mélanges » ou qui se forme, lors de l'utilisation des compositions détergentes, par décomposition de l'hydroperoxyde.

Conditions générales du lavage et mesure de son efficacité :

Les compositions détergentes sont réalisées par pesée des quantités voulues des divers ingrédients et dissoutes dans une eau déminéralisée puis rendue calcaire jusqu'à un taux déterminé par addition de carbonate de calcium (généralement 300 ppm de $CaCO_3$). On utilise pour le lavage un appareil de laboratoire de marque Ahiba « Texomat » type G6B, dont chaque bol est chargé de 6 g de poudre détergente et 600 ml d'eau. Le pH de la solution ainsi obtenue est ajusté à la valeur désirée par addition de traces de soude ; la soude peut également être incorporée dans la poudre au cours de sa préparation par broyage. De préférence, la solution de lavage aura un pH compris entre 9 et 12 et mieux de 11 ± 0,5. Dans tous les exemples ci-dessous il sera question du pH de la solution de détergent avant lavage, étant entendu que pendant le lavage, le pH diminue d'environ 0,5 unité.

On chauffe jusque 30 °C et maintient 15 mn à cette température puis on introduit les paniers porte-

tissus dans les bols et démarre l'agitation. On monte la température de 30 à 90-95 °C pendant 40 mn et maintient pendant 50 mn à 90-95 °C. Les paniers sont trempés dans l'eau courante et les échantillons de tissus rincés à l'eau déminéralisée puis séchés.

Les lavages sont effectués sur des échantillons de tissus-tests EMPA imprégnés des salissures artificielles suivantes : sang, vin rouge, noir Immédial (teinture au noir au soufre) que nous appellerons respectivement ci-dessous : sang, vin, noir.

L'efficacité du lavage par un détergent donné est mesurée à l'aide du système de chromaticité uniforme L, a, b, « cube-root » (1958) qui permet de représenter les écarts de couleurs. Dans ce système (schématiquement représenté à la figure en annexe) L désigne le degré de blancheur « pure » (Luminance) entre le noir absolu (0) et le blanc pur (100), a designe suivant son signe une coloration tendant vers le vert (valeurs négatives) ou vers le rouge (valeurs positives), b une coloration tendant vers le bleu (valeurs négatives) ou vers le jaune (valeurs positives). Dans ce système à trois axes orthonormés, la valeur obtenue pour un échantillon sera donc représentée par un point ; la distance de ce point à un autre, donnant l'écart total de chromaticité entre eux, est telle que :

$$\Delta E = \sqrt{(L_1 - L_2)^2 + (a_1 - a_2)^2 + (b_1 - b_2)^2}$$

En prenant comme référence le point $L_1$, $a_1$, $b_1$ (100,0,0) représentant la blancheur « pure » théorique maximum, l'écart de chromaticité absolu pour l'échantillon mesuré (L, a, b) sera égal à :

$$\Delta E_{abs} = \sqrt{(100 - L)^2 + a^2 + b^2};$$

plus cette valeur est faible, plus le lavage est efficace.

Les valeurs L, a, b, sont données directement par l'appareil de mesure (Colorimètre par réflexion, de marque DUCOLOR NEOTEC-220 L.).

Les exemples non limitatifs suivants illustrent l'invention :

Exemple 1

Peroxydation du diisopropyl-2,4-benzène sulfonate de sodium

On introduit dans le réacteur décrit page quatre 160 g de diisopropylbenzène sulfonate de sodium, 40 ppm (par rapport au sulfonate) de cobalt sous forme de naphténate de cobalt titrant 6 % de cobalt, 480 g d'eau, 20 g d'eau oxygénée à 49,7 % en poids et 3,1 g de carbonate de sodium pour ajuster le pH du milieu réactionnel à 9,5. On fait circuler la phase gazeuse à travers le mélange réactionnel porté à 84 °C en admettant dans cette phase gazeuse de l'oxygène pur. L'absorption de l'oxygène, lente au début, atteint une vitesse d'environ 0,5 g par heure au bout d'environ 4 heures.

Le titre pondéral du mélange réactionnel en hydroperoxyde est de 6,2 % après 10 heures de réaction (rendement en hydroperoxyde par rapport à l'oxygène : 88 %).

On poursuit la réaction pendant encore 6 heures. Le titre en hydroperoxyde du mélange réactionnel est de 7,5 % en poids et le rendement en hydroperoxyde par rapport à l'oxygène de 76 %. Au total, 7,2 g d'oxygène ont été absorbés.

L'évaporation à sec sous vide à un maximum de 40 °C d'une partie du mélange réactionnel fournit un produit sec composé de :

— 59 % de sulfonate de départ,
— 30 % de l'hydroperoxyde du diisopropyl-2,4-benzène sulfonate de sodium,
— 9 % de l'alcool provenant de la décomposition de l'hydroperoxyde.

Le taux de transformation du sulfonate est globalement de 37 % et en hydroperoxyde de 28 %.

Le reste du mélange réactionnel est refroidi à 2 °C et filtré. Le précipité d'une part et le filtrat d'autre part sont séchés sous vide. Les poudres ainsi obtenues en quantités sensiblement équivalentes, ont les compositions suivantes en % en poids :

| | Précipité | Poudre provenant du séchage du filtrat |
|---|---|---|
| sulfonate de départ | 71 % | 51 % |
| Hydroperoxyde | 18 % | 40 % |
| Alcool | 11 % | 7 % |

Exemple 2

Peroxydation du sel de sodium de l'acide méta-cymène sulfonique (acide méthyl-2-isopropyl-4-benzène sulfonique)

On introduit dans le réacteur 112 g du sel de sodium de l'acide méta-cymène sulfonique, 30 ppm (par

rapport au sulfonate) de cobalt sous forme de naphténate contenant 6 % de cobalt, 665 g d'eau, 20 g d'eau oxygénée à 49,7 % en poids et 3 g de carbonate de sodium pour ajuster le pH du milieu à 9,7. La température est portée à 84,5 °C pendant que l'on recycle dans le réacteur la phase gazeuse dans laquelle on introduit de l'oxygène à une vitesse moyenne de 0,36 g/h.

Au bout de 9 h de réaction le titre pondéral du mélange réactionnel en hydroperoxyde est de 1,9 % (rendement en hydroperoxyde par rapport à l'oxygène : 92 %).

On arrête la réaction après 21 heures au total. Il a été consommé 5,06 g d'oxygène. A ce moment le titre pondéral du mélange réactionnel en hydroperoxyde est de 4 % (rendement en hydroperoxyde par rapport à l'oxygène : 77 %).

On prélève une partie du milieu réactionnel que l'on sèche sous vide. Le produit sec obtenu contient :

— 63 % de sulfonate de départ,
— 27,5 % d'hydroperoxyde correspondant,
— 8 % d'alcool provenant de la décomposition de l'hydroperoxyde.

Le taux de conversion global du sulfonate est de 33 % ; son taux de conversion en hydroperoxyde de 25,5 %.

Le reste de la masse réactionnelle est refroidi à 20 °C et filtré. Le précipité obtenu, d'une part, et le filtrat, d'autre part, sont séchés sous vide à 40 °C maximum. Le précipité représente environ 1/3 de la quantité totale de produits secs obtenue.

L'analyse de ces produits secs donne les compositions suivantes (% en poids) :

| | Précipité | Poudre sèche venant du filtrat |
|---|---|---|
| Sulfonate | 86 % | 50 % |
| Hydroperoxyde | 10 % | 39 % |
| Alcool | 4 % | 10 % |

Exemple 3

Peroxydation du sel de sodium de l'acide para-cymène sulfonique (acide méthyl-2-isopropyl-5-benzène sulfonique)

On introduit dans le réacteur 195 g du sel de sodium de sodium de l'acide para-cymène sulfonique, 17 ppm (par rapport au sulfonate) de cobalt sous forme de naphténate contenant 6 % de cobalt, 565 g d'eau, 20 g d'eau oxygénée à 49,7 % et 3 g de carbonate de sodium pour ajuster le pH du milieu à 9,7. On démarre le recyclage de la phase gazeuse à travers le masse réactionnelle pendant que l'on monte la température de celle-ci à 85 °C. On introduit de l'oxygène dans le courant de gaz à un débit moyen de 0,88 g/h.

Après 8 h de réaction le titre pondéral du mélange réactionnel en hydroperoxyde est de 4,7 % (rendement en hydroperoxyde par rapport à l'oxygène : 93 %).

On arrête la réaction après un total de 16 h. Le titre pondéral du mélange réactionnel en hydroperoxyde est de 6,3 % ; la quantité totale d'oxygène absorbé est de 7,4 g et le rendement en hydroperoxyde par rapport à l'oxygène de 79 %.

Une partie du mélange réactionnel est séchée sous vide et donne un produit sec dont la composition est la suivante (% en poids) :

— sulfonate de départ : 67 %
— hydroperoxyde : 25 %
— alcool : 6 %

Le taux de transformation global du sulfonate est de 28 % et le taux de transformation en hydroperoxyde de 22 %.

On refroidit le reste du mélange réactionnel à 2 °C, filtre et sèche d'une part le précipité et d'autre part le filtrat à 20 °C sous vide. Les poudres sont obtenues dans le rapport pondéral 2/3 (du précipité)/1/3 (du filtrat).

Les produits secs obtenus ont les compositions suivantes en % en poids :

| | Précipité | Poudre provenant du filtrat |
|---|---|---|
| Sulfonate | 79 % | 45 % |
| Hydroperoxyde | 18 % | 38 % |
| Alcool | 2 % | 15 % |

Exemple 4

Peroxydation du mélange de sels de sodium des acides méta et para-cymène sulfoniques

On introduit dans le réacteur :

146 g de sel de sodium de l'acide méta-cymène sulfonique,

79 g de sel de sodium de l'acide para-cymène sulfonique,

30 ppm (par rapport au mélange des sulfonates) de cobalt sous forme de naphténate à 6 % de cobalt,

710 g d'eau,

20 g d'eau oxygénée à 49,7 %, et

3 g de carbonate de sodium pour ajuster le pH du milieu à 9,6.

On recycle la phase gazeuse à travers le mélange réactionnel et porte celui-ci à 85 °C ; on introduit de l'oxygène à un débit moyen de 0,84 g/h.

Après 14 h de réaction, le mélange réactionnel a un titre pondéral en hydroperoxyde de 7,8 % (rendement par rapport à l'oxygène : 92 %).

On arrête la réaction après un total de 20 heures. Le titre pondéral du mélange réactionnel en hydroperoxyde est de 9,5 % ; 12,9 g d'oxygène ont été, au total, absorbés : le rendement en hydroperoxyde par rapport à l'oxygène est de 84,5 %.

Une partie du mélange réactionnel est séchée sous vide à 40 °C maximum.

Sa composition est la suivante, en % en poids :

— sulfonate de départ : 52 %
— hydroperoxyde : 39 %
— alcool : 7 %

Le taux de transformation global du mélange des sulfonates est de 42 %, le taux de transformation en hydroperoxyde de 36 %.

Le reste du milieu réactionnel est débarrassé, sous vide, de 10% de l'eau présente, refroidi à 20 °C où il a l'aspect d'un gel et filtré à cette température.

On obtient un précipité et un filtrat que l'on sèche séparément sous vide. Les produits secs recueillis (rapport pondéral 1/1) ont les compositions suivantes en % en poids :

| | Précipité | Poudre provenant du filtrat |
|---|---|---|
| Sulfonate | 69 % | 36 % |
| Hydroperoxyde | 27 % | 52 % |
| Alcool | 3 % | 11 % |

Exemple 5

Peroxydation du sel de sodium de l'acide cumène sulfonique (acide para isoporopyl benzène sulfonique)

On introduit dans le réacteur 185 g du sel de sodium de l'acide cumène sulfonique, 10 ppm de cobalt (par rapport au sulfonate) sous forme de naphténate à 6 % de cobalt, 500 g d'eau, 18 g du mélange solide et sec d'hydroperoxyde du sel de sodium de l'acide cumène sulfonique et de sel de sodium de l'acide cumène sulfonique, ledit mélange provenant d'une opération précédente (précipité obtenu par concentration à sec puis reprise par le méthanol, comme décrit ci-dessous) et contenant environ 30 % d'hydroperoxyde, et 0,5 g de carbonate de sodium pour ajuster le pH du milieu à 10. On met en route le recyclage de la phase gazeuse et porte le mélange réactionnel à 85 °C. On introduit dans le courant gazeux de l'oxygène à un débit moyen de 1,2 g/h.

Après 10 h de réaction le titre pondéral en hydroperoxyde du mélange réactionnel est de 12 %, le rendement par rapport à l'oxygène de 90 % (86 % si l'on tient compte de l'hydroperoxyde introduit au départ) et la quantité totale d'oxygène absorbé de 12 g.

On concentre à sec le mélange réactionnel sous vide à 40 °C maximum et on obtient une poudre titrant, en poids :

— sulfonate de départ : 48 %
— hydroperoxyde : 40 %
— alcool de décomposition : 11 %

Le taux de transformation du sulfonate est globalement de 48 % ; le taux de transformation en hydroperoxyde de 36 %.

Une partie en poids de cette poudre est mélangée à 2 parties en poids de méthanol et le mélange agité pendant quelques minutes à 40 °C. On refroidit à 5 °C et filtre le précipité obtenu. Le précipité d'une part et le filtrat d'autre part sont séchés sous vide à 20 °C. On obtient ainsi un précipité qui titre 30 % en poids d'hydroperoxyde et une poudre (provenant du filtrat) qui titre 50 % en poids d'hydroperoxyde.

Exemples d'application des compositions selon l'invention, contenant un hydroperoxyde d'un mono- ou polyalkylarylsulfonate de sodium, à la préparation de compositions détergentes :

Les tissus tests utilisés dans les exemples ci-dessous ont, à l'origine, à l'état sali, les valeurs L, a, b, $\Delta E_{abs}$ suivantes :

TABLEAU I

| | L | a | b | $\Delta E_{abs}$ |
|---|---|---|---|---|
| Sang | 45,1 | + 15 | + 7,1 | 57,4 |
| Vin | 71,7 | + 9,5 | + 6,9 | 30,6 |
| Noir | 70,6 | − 0,06 | − 1,9 | 29,5 |

On utilisera les abréviations suivantes :
Dodécylbenzène sulfonate de sodium, à environ 50 % de matière active : DDBS
Sel de sodium de suif : Savon
Détergent non ionique : DNI
Tripolyphosphate de sodium : TPP
Silicate de sodium, à environ 44 % de matière active : SiS
Perborate de sodium tétrahydré ($NaBO_3$, $4H_2O$) : PBS
Acide nitrilotriacétique : NTA
Sel de sodium de l'acide Ethylène-diaminetétracétique : EDTA
Sulfate de sodium : SNa

Exemple 6 (comparatif)

Lavage sans agent de blanchiment

On utilise dans les conditions opératoires décrites ci-dessus page 9 une poudre composée de (en % en poids) :

| | |
|---|---|
| DDBS | 10 % |
| Savon | 7 % |
| DNI | 6 % |
| TPP | 40 % |
| SiS | 10 % |
| SNa | 27 % |

La solution de lavage (1 partie de poudre pour 100 parties d'eau) a un pH de 10, ajusté par une faible quantité de soude.

On obtient les résultats suivants :

TABLEAU II

| | L | a | b | $\Delta E_{abs}$ |
|---|---|---|---|---|
| Sang | 87 | 0,6 | 7,7 | 15,1 |
| Vin | 88 | 2 | 6,9 | 14,0 |
| Noir | 73,6 | − 0,1 | − 2,4 | 26,5 |

Ainsi sans agent de blanchiment le noir n'est pratiquement pas lavé, alors que sang et vin voient leur composante L augmenter (nettement dans le cas du sang), leur composante rouge fortement diminuer, leur composante jaune rester pratiquement inchangée.

Exemple 7 (comparatif)

Lavage à l'aide d'une lessive classique contenant du perborate de sodium

On utilise pour le lavage à pH 10 une poudre composée de (en % en poids) :

| | |
|---|---|
| DDBS | 10 % |
| Savon | 7 % |
| DNI | 6 % |
| TPP | 40 % |
| SiS | 10 % |
| EDTA | 1 % |
| PBS | 26 % |

On obtient les résultats suivants :

TABLEAU III

| | L | a | b | $\Delta E_{abs}$ |
|---|---|---|---|---|
| Sang | 90,3 | – 0,7 | 13,2 | 16,4 |
| Vin | 93,8 | – 0,7 | 2,8 | 6,8 |
| Noir | 81,8 | – 1,2 | – 0,9 | 18,3 |

Par rapport à la lessive sans perborate on note donc un lavage nettement meilleur du vin, meilleur du noir mais légèrement moins bon pour le sang, avec accroissement de la composante jaune. On note également dans le cas du sang un certain durcissement du tissu après lavage.

Le lavage à pH 12 est plus efficace pour le noir et nettement plus pour le sang comme indiqué ci-dessous :

TABLEAU IV

| | L | a | b | $\Delta E_{abs}$ |
|---|---|---|---|---|
| Sang | 92,2 | – 0,1 | 7,1 | 10,5 |
| Vin | 94,2 | – 0,6 | 2,4 | 6,3 |
| Noir | 83,3 | – 1,2 | – 0,9 | 16,8 |

Si on ajoute à la composition détergente 100 ppm de cuivre sous forme de $CuSO_4$, $5H_2O$ (cette quantité correspond à 1 ppm de $Cu^{++}$ dans la solution de lavage), on obtient les nouvelles valeurs, pour un lavage à pH 12 :

TABLEAU V

| | L | a | b | $\Delta E_{abs}$ |
|---|---|---|---|---|
| Sang | 86,8 | 0,04 | 8,2 | 15,5 |
| Vin | 88,5 | 2,1 | 8,5 | 14,4 |
| Noir | 79,7 | – 0,7 | – 0,8 | 20,3 |

On note donc un effet néfaste de l'addition de cuivre dans les poudres détergentes au perborate bien que ces poudres contiennent de l'EDTA.

La diminution du taux de perborate (de manière à obtenir un taux d'oxygène actif équivalent à celui de 5,3 % d'hydroperoxyde pur suivant l'invention) conduit à des résultats légèrement moins bons comme le montre le tableau VI. La poudre de lavage contenait :

| | |
|---|---|
| DDBS | 10 % |
| Savon | 7 % |
| DNI | 6 % |
| TPP | 40 % |
| SiS | 10 % |
| EDTA | 1 % |
| PBS | 3,2 % |
| SNa | 22,8 % |

TABLEAU VI (lavage à pH 10)

| | L | a | b | $\Delta E_{abs}$ |
|---|---|---|---|---|
| Sang | 88,9 | 0,9 | 13,9 | 17,8 |
| Vin | 92,8 | – 0,4 | 3,8 | 8,1 |
| Noir | 76,7 | – 1 | – 2 | 23,4 |

Si l'on supprime l'EDTA de cette formulation, la valeur $\Delta E_{abs}$ diminue d'environ 1 point dans les trois cas.

Exemples 8 à 22

Lavages avec les compositions détergentes suivant l'invention

Dans tous ces exemples on a utilisé comme agent de blanchiment l'hydroperoxyde du cumène sulfonate de sodium à l'état de mélange avec le cumène sulfonate de sodium et l'alcool provenant de la décomposition de l'hydroperoxyde, ledit mélange, qui est une poudre sèche, contenant respectivement en poids 50 %, 40 %, 10 % des trois composés ci-dessus.

C'est la teneur en ce mélange (qui sera appelé HPC) qui figurera dans le tableau VII ci-dessous.

Les lavages sont effectués dans les conditions précédemment décrites.

Le pH du milieu est ajusté en incorporant de la soude finement broyée aux compositions détergentes.

Les caractéristiques de ces compositions figurent au tableau VII et les résultats obtenus au tableau VIII.

L'exemple n° 22 a été réalisé en remplaçant poids pour poids le sulfate de sodium par le carbonate de sodium ($Na_2CO_3$) par rapport à l'exemple 19.

Le remplacement du perborate de sodium par une quantité équivalente (pouvoir oxydant égal) de « mélange » (H.P.C.) procure une amélioration notamment pour le lavage des taches de sang, l'addition de sulfate de cuivre améliore en outre le lavage du « noir » et l'addition de NTA améliore en outre le lavage des taches de vin.

On notera une certaine influence du pH sur les résultats obtenus pour le lavage en particulier des taches de sang et de noir.

Exemple 23

A la place de l'hydroperoxyde du cumène sulfonate de sodium, on utilise comme agent de blanchiment le monohydroperoxyde du métadiisopropylbenzène sulfonate de sodium.

Le produit obtenu par peroxydation du métadiisopropylbenzène sulfonate de sodium contient en % en poids :

hydroperoxyde : 40 %
produit de départ non transformé : 50 %
alcool provenant de la décomposion de l'hydroperoxyde : 10 %

Ce mélange (HPDP) est incorporé dans une poudre détergente de composition suivante (en % en poids) :

| | | | |
|---|---|---|---|
| DDBS | 10 % | PBS | 2 % |
| Savon | 7 % | HPDP | 10,7 % |
| DNI | 6 % | NTA | 0,5 % |
| TPP | 40 % | SNa | 13,8 % |
| SiS | 10 % | Cu | 100 ppm (sous forme de $CuSO_4$, $5H_2O$). |

Cette poudre est dissoute dans 100 fois son poids d'eau et le pH de la solution est ajusté à 12 par addition de traces de soude.

Le lavage est effectué dans les conditions énoncées ci-dessus. Il conduit aux résultats suivants :

| | L | a | b | $\Delta E_{abs}$ |
|---|---|---|---|---|
| Sang | 92,3 | − 0,3 | 5,9 | 9,7 |
| Vin | 93 | − 0,1 | 4,8 | 8,5 |
| Noir | 81,2 | − 0,5 | − 1,4 | 18,8 |

TABLEAU VII — Compositions détergentes solides utilisées

| composant en % en poids / Ex. | DDBS | Savon | DNI | TPP | SiS | PBS | HPC | NTA | EDTA | SNa | $Cu^{++}$ en ppm | pH du milieu lessiviel |
|---|---|---|---|---|---|---|---|---|---|---|---|---|
| 8 | — | 7 | 6 | 40 | 10 | — | 26,5 | — | — | 10,5 | 400 | 11 |
| 9 | 10 | 7 | 6 | 40 | 10 | — | 10 | — | — | 17 | 0 | 10,5 |
| 10 | 10 | 7 | 6 | 40 | 10 | — | 10 | — | — | 17 | 0 | 11,5 |
| 11 | 10 | 7 | 6 | 40 | 10 | — | 10 | — | — | 17 | 100 | 11,5 |
| 12 | 10 | 7 | 6 | 40 | 10 | 2 | 4 | — | — | 21 | 0 | 10 |
| 13 | 10 | 7 | 6 | 40 | 10 | 2 | 4 | — | — | 21 | 400 | 10 |
| 14 | 10 | 7 | 6 | 40 | 10 | 2 | 4 | — | — | 21 | 100 | 11 |
| 15 | 10 | 7 | 6 | 40 | 10 | 2 | 4 | — | — | 21 | 100 | 12 |

TABLEAU VII — Suite

| Ex. / composant en % en poids | DDBS | Savon | DNI | TPP | SiS | PBS | HPC | NTA | EDTA | SNa | Cu⁺⁺ en ppm | pH du milieu lessiviel |
|---|---|---|---|---|---|---|---|---|---|---|---|---|
| 16 | 10 | 7 | 6 | 40 | 10 | 2 | 4 | 3,9 | — | 17,1 | 200 | 12 |
| 17 | 10 | 7 | 6 | 40 | 10 | 2 | 4 | 0,5 | — | 20,5 | 100 | 11 |
| 18 | 10 | 7 | 6 | 40 | 10 | 2 | 4 | 1 | — | 20 | 100 | 12 |
| 19 | 10 | 7 | 6 | 40 | 10 | 2 | 8 | 0,5 | — | 16,5 | 100 | 11 |
| 20 | 10 | 7 | 6 | 40 | 10 | 2 | 8 | 0,5 | — | 16,5 | 100 | 12 |
| 21 | 10 | 7 | 6 | 40 | 10 | 2 | 4 | — | 1 | 20 | 800 | 10 |
| 22 | 10 | 7 | 6 | 40 | 10 | 2 | 8 | 0,5 | — | $NaCO_3$ 16,5 | 100 | 11 |

TABLEAU VIII — Résultats des lavages

| Salissures | SANG | | | | VIN | | | | NOIR | | | |
|---|---|---|---|---|---|---|---|---|---|---|---|---|
| Ex. | L | a | b | $\Delta E_{abs}$ | L | a | b | $\Delta E_{abs}$ | L | a | b | $\Delta E_{abs}$ |
| 8 | non mesuré | | | | non mesuré | | | | 85,8 | − 0,6 | 1,2 | 14,3 |
| 9 | 89,2 | 0,7 | 10,7 | 15,2 | 88,9 | 1,5 | 6,5 | 12,1 | 74,7 | 0,04 | − 2,1 | 25,4 |
| 10 | 92 | − 0,2 | 6,9 | 10,6 | 88 | 1,8 | 6,8 | 13,9 | 77 | − 0,1 | − 2 | 23,1 |
| 11 | 91,5 | − 0,05 | 7,2 | 11,1 | non mesuré | | | | 84,5 | − 0,3 | − 0,1 | 15,5 |
| 12 | 89,8 | 0,5 | 11 | 15 | 93,6 | − 0,34 | 3,6 | 7,3 | 75,8 | − 0,6 | − 2,2 | 24,3 |
| 13 | 87,2 | 0,5 | 7,7 | 14,9 | 87,5 | 2,5 | 8 | 15 | 77,5 | 0,5 | − 1,4 | 22,5 |
| 14 | 89,6 | 0,3 | 7 | 12,5 | 87,6 | 2,6 | 7,7 | 14,8 | 78,5 | − 0,4 | − 1,2 | 21,5 |
| 15 | 90,7 | − 0,2 | 6 | 11,1 | 87,5 | 2,5 | 7,3 | 14,7 | 82,9 | − 0,7 | − 0,2 | 17,1 |
| 16 | 90,1 | − 0,04 | 7 | 12,1 | 93,8 | − 0,4 | 3,3 | 7 | 78,8 | − 0,8 | − 1,7 | 21,3 |
| 17 | 90,3 | 0,2 | 8,3 | 12,8 | 93,8 | − 0,4 | 3,5 | 7,1 | 78,9 | − 0,7 | − 1,7 | 21,3 |
| 18 | 91,2 | − 0,1 | 6,3 | 10,8 | 93,7 | − 0,4 | 3,8 | 7,4 | 81,6 | − 0,8 | − 0,7 | 18,4 |
| 19 | 92,5 | − 0,3 | 6,6 | 10 | 93,8 | − 0,4 | 3,3 | 7 | 79,7 | − 0,7 | − 1,6 | 20,4 |
| 20 | 92 | − 0,3 | 6 | 10 | 93,1 | − 0,2 | 4,7 | 8,3 | 84 | − 0,9 | 0,4 | 16 |
| 21 | 90,2 | 0,25 | 9,1 | 13,4 | 92,7 | − 0,03 | 4,6 | 8,6 | 76,7 | − 0,8 | − 1,9 | 23,4 |
| 22 | 92,9 | − 0,3 | 6,3 | 9,5 | 93,4 | − 0,3 | 3,4 | 7,4 | 80 | − 0,8 | 1,5 | 20 |

**Revendications**

1. Composition comprenant un mono- ou polyalkylarylsulfonate de métal alcalin de formule :

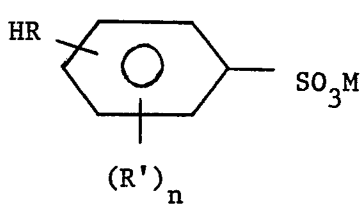

dans laquelle M est un métal alcalin, RH est un radical alkyle inférieur, R′ est un radical alkyle inférieur, n est un nombre entier inférieur à 5, et un hydroperoxyde de formule :

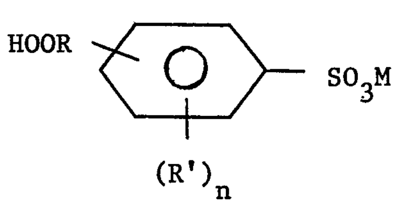

dans laquelle M, RH, R' et n ont les mêmes significations que ci-dessus, caractérisée en ce qu'elle se présente à l'état solide et sec et en ce qu'elle contient :

— de 5 à 75 % en poids dudit mono- ou polyalkylarylsulfonate de métal alcalin,

— de 25 à 70 % en poids dudit hydroperoxyde,

— de 0 à 30 % en poids de l'alcool provenant de la décomposition dudit hydroperoxyde et ayant la formule :

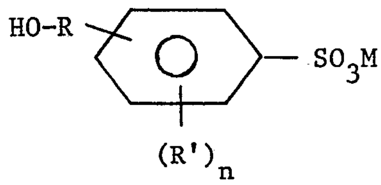

dans laquelle M, RH, R' et n ont les mêmes significations que ci-dessus.

2. Composition selon la revendication 1, caractérisée en ce que le mono- ou polyalkylarylsulfonate de métal alcalin est choisi parmi les sels de sodium des acides para-cumène sulfonique, cymène sulfonique et diisopropyl benzène sulfonique.

3. Procédé de peroxydation d'un mono- ou polyalkylarylsulfonate de métal alcalin de formule :

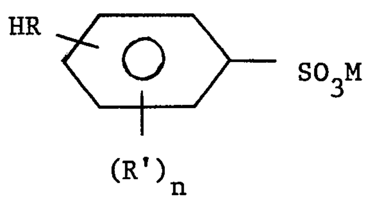

dans laquelle M est un métal alcalin, RH est un radical alkyle inférieur, R' est un radical alkyle inférieur, n est un nombre entier inférieur à 5, par réaction, en solution aqueuse, avec de l'oxygène, caractérisé en ce que :

— la réaction est réalisée à une température non supérieure à 85 °C,

— la réaction est effectuée à pH compris entre 9 et 10,5,

— on utilise comme catalyseur de 1 à 50 ppm de cobalt, par rapport au mono- ou polyalkylarylsulfonate de métal alcalin.

4. Procédé selon la revendication 3 caractérisé en ce que le cobalt est sous la forme de naphténate de cobalt.

5. Procédé selon l'une des revendications 3 et 4, caractérisé en ce que la réaction de peroxydation est réalisée en présence de peroxyde.

6. Procédé selon la revendication 5, caractérisé en ce que le peroxyde est de l'eau oxygénée utilisée à raison de 5 à 10 parties en poids (calculé en eau oxygénée pure) pour 100 parties en poids de mono- ou polyalkylarylsulfonate de métal alcalin.

7. Procédé pour la préparation de composition selon l'une des revendications 1 et 2, caractérisé en ce que, après peroxydation en solution aqueuse du mono- ou polyalkylarylsulfonate de métal alcalin selon l'une des revendications 3 à 6, on traite le mélange aqueux obtenu de façon à provoquer une précipitation partielle des produits qu'il contient, que l'on sépare le précipité obtenu, que l'on soumet le cas échéant le filtrat à au moins une cristallisation supplémentaire, et que l'on évapore le filtrat à sec à une température au plus égale à 40 °C.

8. Procédé selon la revendication 7, caractérisé en ce que ladite précipitation est réalisée par refroidissement, entre 0 et 20 °C, de ladite solution aqueuse.

9. Procédé selon la revendication 7, caractérisé en ce que ladite précipitation est réalisée par concentration sous vide à température au plus égale à 40 °C suivie d'un refroidissement à température comprise entre 2 et 20 °C.

10. Procédé selon la revendication 7, caractérisé en ce que le mélange réactionnel est transformé en suspension par concentration à sec sous vide et reprise du produit obtenu, par du méthanol.

11. Compositions détergentes caractérisées en ce qu'elles contiennent, en tant qu'agent de blanchiment, un hydroperoxyde d'un mono- ou polyalkylarylsulfonate de métal alcalin de formule :

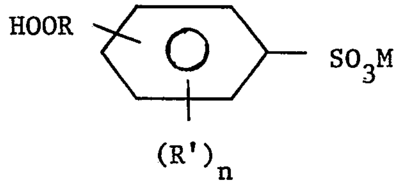

dans laquelle M est un métal alcalin, RH est un radical alkyle inférieur, R' est un radical alkyle inférieur, n est un nombre entier inférieur à 5.

12. Compositions détergentes sèches, caractérisées en ce qu'elles contiennent jusqu'à 30 % en poids d'une composition selon l'une des revendications 1 et 2.

13. Compositions détergentes selon l'une des revendications 11 et 12, caractérisées en ce qu'elles contiennent également du perborate de sodium.

14. Compositions détergentes selon l'une des revendications 11 à 13, caractérisées en ce qu'elles contiennent jusqu'à 1 000 ppm en poids de cuivre sous forme d'un sel cuivrique soluble dans l'eau.

15. Compositions détergentes selon la revendication 14, caractérisées en ce qu'elles contiennent de 0,1 à 5 % en poids d'au moins un agent complexant du cuivre choisi parmi l'acide nitrilotriacétique, l'acide éthylène diamine tétracétique et les sels alcalins desdits acides.

16. Compositions détergentes selon l'une des revendications 11 à 15, caractérisées en ce qu'elles contiennent une quantité suffisante d'une base pour que les solutions aqueuses desdites compositions aient un pH de l'ordre de 9 à 12.

17. Compositions détergentes selon l'une des revendications 11 à 16, caractérisées en ce que l'hydroperoxyde d'un mono- ou polyalkylarylsulfonate de métal alcalin est l'hydroperoxyde de l'isopropyl benzène sulfonate de sodium.

18. Compositions détergentes selon l'une des revendications 11 à 16, caractérisées en ce que l'hydroperoxyde d'un mono- ou polyalkylarylsulfonate de métal alcalin est le monohydroperoxyde du diisopropyl benzène sulfonate de sodium.

**Claims**

1. Composition comprising an alkali metal mono- or polyalkylarylsulfonate of formula :

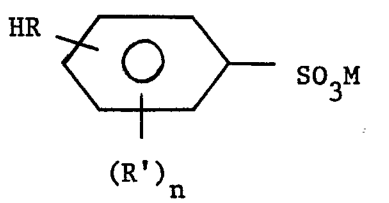

in which M is an alkali metal, RH is a lower alkyl radical, R' is a lower alkyl radical, n is a whole number less than 5, and a hydroperoxide of formula :

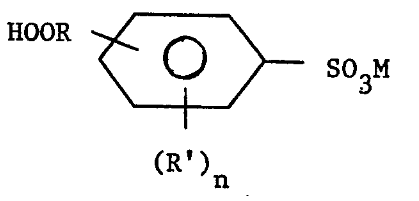

in which M, RH, R' and n are as given, above, characterised in that it is in a solid and dry state and in that it contains :

— from 5 to 75 % by weight of the said alkali metal mono- or polyalkylarylsulfonate,

— from 25 to 70 % by weight of the said hydroperoxide,

— from 0 to 30 % by weight alcohol coming from the decomposition of said hydroperoxide and having the formula :

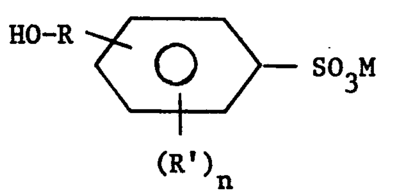

in which M, RH, R' and n are as given above.

2. Composition according to claim 1, characterised in that the alkali metal mono- or polyalkylarylsulfonate is chosen from the sodium salts of para-cumene sulfonic acid, cymene sulfonic acid and diisopropyl benzene sulfonic acid.

3. Method for the peroxidation of an alkali metal mono- or polyalkylarylsulfonate of formula :

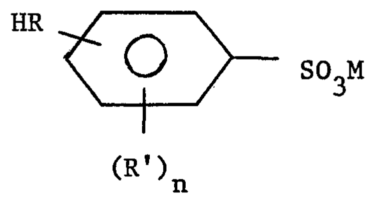

in which M is an alkali metal, RH is a lower alkyl radical, R' is a lower alkyl radical, n is a whole number less than 5, by reaction, in an aqueous solution, with oxygen, characterised in that:
— the reaction is carried out at a temperature no higher than 85 °C,
— the reaction is carried out at a pH of between 9 and 10.5,
— 1 to 50 ppm cobalt, with respect to the alkali metal mono- or polyalkylarylsulfonate, are used as a catalyst.

4. Method according to claim 3, characterised in that the cobalt is in the form of cobalt naphthenate.

5. Method according to one of claims 3 and 4, characterised in that the peroxidation reaction is carried out in the presence of peroxide.

6. Method according to claim 5, characterised in that the peroxide is hydrogen peroxide used at the rate of 5 to 10 parts by weight (calculated as pure hydrogen peroxide) for 100 parts by weight alkali metal mono- or polyalkylsulfonate.

7. Method for the preparation of the composition according to one of claims 1 and 2, characterised in that, after peroxidation in an aqueous solution of the alkali metal mono- or polyalkylarylsulfonate according to one of claims 3 to 6, the aqueous mixture obtained is treated in order to cause partial precipitation of the products which it contains, that the precipitate obtained is separated, that if necessary the filtrate is subjected to at least one additional crystallization, and that the filtrate is evaporated to dryness at a temperature at the most equal to 40 °C.

8. Method according to claim 7, characterised in that said precipitation is carried out by cooling said aqueous solution to between 0 and 20 °C.

9. Method according to claim 7, characterised in that said precipitation is carried out by concentration under vacuum at a temperature at the most equal to 40 °C, followed by cooling to a temperature of between 2 and 20 °C.

10. Method according to claim 7, characterised in that the reaction mixture is transformed into a suspension by concentration to dryness under vacuum and by taking up the product obtained in methanol.

11. Detergent compositions, characterised in that they contain, as a bleaching agent, a hydroperoxide of an alkali metal mono- or polyalkylarylsulfonate of formula:

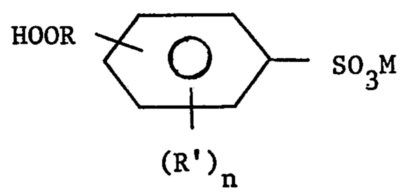

in which M is an alkali metal, RH is a lower alkyl radical, R' is a lower alkyl radical, n is a whole number less than 5.

12. Dry detergent compositions, characterised in that they contain up to 30 % by weight of a composition according to one of claims 1 and 2.

13. Detergent compositions according to one of claims 11 and 12, characterised in that they also contain sodium perborate.

14. Detergent compositions according to one of claims 11 to 13, characterised in that they contain up to 1 000 ppm by weight copper in the form of a water-soluble copper salt.

15. Detergent compositions according to claim 14, characterised in that they contain from 0.1 to 5 % by weight of at least one agent for complexing copper chosen from nitrilotriacetic acid, ethylenediaminetetraacetic acid and the alkali metal salts of said acids.

16. Detergent compositions according to one of claims 11 to 15, characterised in that they contain a sufficient quantity of a base in order that the aqueous solutions of said compositions have a pH of the order of 9 to 12.

17. Detergent compositions according to one of claims 11 to 16, characterised in that the hydroperoxide of an alkali metal mono- or polyalkylarylsulfonate is the hydroperoxide of sodium isopropyl benzene sulfonate.

18. Detergent compositions according to one of claims 11 to 16, characterised in that the

hydroperoxide of an alkali metal mono- or polyalkylarylsulfonate is the mono hydroperoxide of sodium diisopropyl benzne sulfonate.

**Ansprüche**

1. Zusammensetzung mit einem Alkali-mono- oder -polyalkylarylsulfonat der Formel

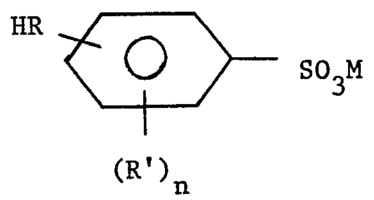

in der bedeuten M ein Alkalimetall, RH eine niedere Alkylgruppe, R' eine niedere Alkylgruppe und n eine ganze Zahl unter 5, und einem Hydroperoxid der Formel

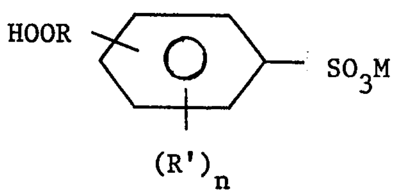

mit M, RH, R' und n wie oben, dadurch gekennzeichnet, daß sie in festem, trockenem Zusatz vorliegt und

— 5 bis 75 Gew.-% des Alaki-mono- oder -polyalkylarylsulfonats,
— 25 bis 70 Gew.-% des Hydroperoxids und
— 0 bis 30 Gew.-% des bei der Zersetzung des Hydroperoxids entstehenden Alkohols der Formel

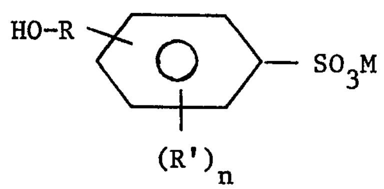

mit M, RH, R' und n wie oben enthält.

2. Zusammensetzung nach Anspruch 1, dadurch gekennzeichnet, daß das Alkali-mono- oder -polyalkylarylsulfonat unter den Natriumsalzen von p-Cumolsulfonsäure, Cymolsulfonsäure und Diisopropylbenzolsulfonsäure ausgewählt ist.

3. Verfahren zur Peroxydierung von Alkali-mono- oder -polyalkylarylsulfonaten der Formel

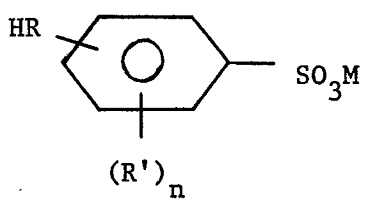

in der bedeuten M ein Alkalimetall, RH eine niedere Alkylgruppe, R' eine niedere Alkylgruppe und n eine ganze Zahl unter 5, durch Umsetzung mit Sauerstoff in wäßriger Lösung, dadurch gekennzeichnet, daß

— die Reaktion bei einer Temperatur $\leq 85\,°C$ und bei einem pH-Wert zwischen 9 und 10,5 durchgeführt und
— als Katalysator 1 bis 50 ppm Kobalt, bezogen auf das Alkali-mono- oder -polyalkylarylsulfonat, verwendet wird.

4. Verfahren nach Anspruch 3, dadurch gekennzeichnet, daß das Kobalt in Form von Kobaltnaphthenat eingesetzt wird.

5. Verfahren nach Anspruch 3 oder 4, dadurch gekennzeichnet, daß die Peroxidierungsreaktion in Gegenwart von Peroxid durchgeführt wird.

6. Verfahren nach Anspruch 5, dadurch gekennzeichnet, daß als Peroxid Wasserstoffperoxid verwendet wird, das in einer Menge von 5 bis 10 Gew.-Teilen (berechnet als reines Wasserstoffperoxid) auf 100 Gew.-Teile Alkali-mono- bzw -polyalkylarylsulfonat eingesetzt wird.

7. Verfahren zur Herstellung der Zusammensetzung nach Anspruch 1 oder 2, dadurch gekennzeichnet, daß nach der Peroxidierung des Alkali-mono- oder -polyalkylarylsulfonats in wäßriger Lösung nach einem der Ansprüche 3 bis 6 das erhaltene wäßrige Gemische so behandelt wird, daß eine partielle Fällung der darin enthaltenen Produkte erzielt wird, der erhaltene Niederschlag abgetrennt wird, das Filtrat erforderlichenfalls mindestens einer zusätzlichen Kristallisation unterzogen und das Filtrat bei einer Temperature ≤ 40 °C zur Trockne eingedampft wird.

8. Verfahren nach Anspruch 7, dadurch gekennzeichnet, daß die Fällung durch Abkühlen der wäßrigen Lösung zwischen 0 und 20 °C vorgenommen wird.

9. Verfahren nach Anspruch 7, dadurch gekennzeichnet, daß die Fällung durch Aufkonzentrieren im Vakuum bei einer Temperatur ≤ 40 °C und anschließendes Abkühlen auf eine Temperatur zwischen 2 und 20 °C durchgeführt wird.

10. Verfahren nach Anspruch 7, dadurch gekennzeichnet, daß das Reaktionsgemisch durch Eindampfen in Vakuum zur Trockne in eine Suspension übergeführt und das erhaltene Produkt in Methanol wiederaufgenommen wird.

11. Reinigungsmittel, dadurch gekennzeichnet, daß sie als Bleichmittel ein Hydroperoxid eines Alkali-mono- oder -polyalkylarylsulfonats der Formel

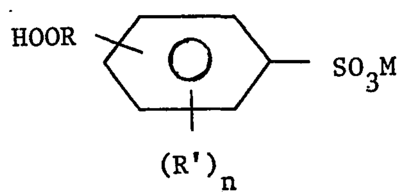

enthalten, in der bedeuten M ein Alkalimetall, RH eine niedere Alkylgruppe, R' eine niedere Alkylgruppe und n eine ganze Zahl unter 5.

12. Trockene Reinigungsmittel, dadurch gekennzeichnet, daß sie bis zu 30 Gew.-% einer Zusammensetzung nach Anspruch 1 oder 2 enthalten.

13. Reinigungsmittel nach Anspruch 11 oder 12, dadurch gekennzeichnet, daß sie ferner Natriumperborat enthalten.

14. Reinigungsmittel nach einem der Ansprüche 11 bis 13, dadurch gekennzeichnet, daß sie bis zu 1 000 ppm (gewichtsbezogen) Kupfer in Form eines wasserlöslichen Kupfer(II) salzes enthalten.

15. Reinigungsmittel nach Anspruch 14, dadurch gekennzeichnet, daß sie 0,1 bis 5 Gew.-% mindestens eines unter Nitrilotriessigsäure, Äthylendiamintetraessigsäure und den Alkalisalzen dieser Säuren ausgewählten Kupferkomplex bildners enthalten.

16. Reinigungsmittel nach einem der Ansprüche 11 bis 15, dadurch gekennzeichnet, daß sie eine ausreichende Menge einer Base zur Einstellung des pH-Werts der wäßrigen Lösungen der Reinigungsmittel im Bereich von etwa 9 bis 12 enthalten.

17. Reinigungsmittel nach einem der Ansprüche 11 bis 16, dadurch gekennzeichnet, daß sie als Hydroperoxid eines Alkali-mono- oder -polyalkylarylsulfonats das Hydroperoxid von Natriumisopropylbenzolsulfonat enthalten.

18. Reinigungsmittel nach einem der Ansprüche 11 bis 16, dadurch gekennzeichnet, daß sie als Hydroperoxid eines Alkali-mono- oder -polyalkylarylsufonats das Monohydroperoxid von Natriumdiisopropylbenzolsulfonat enthalten.

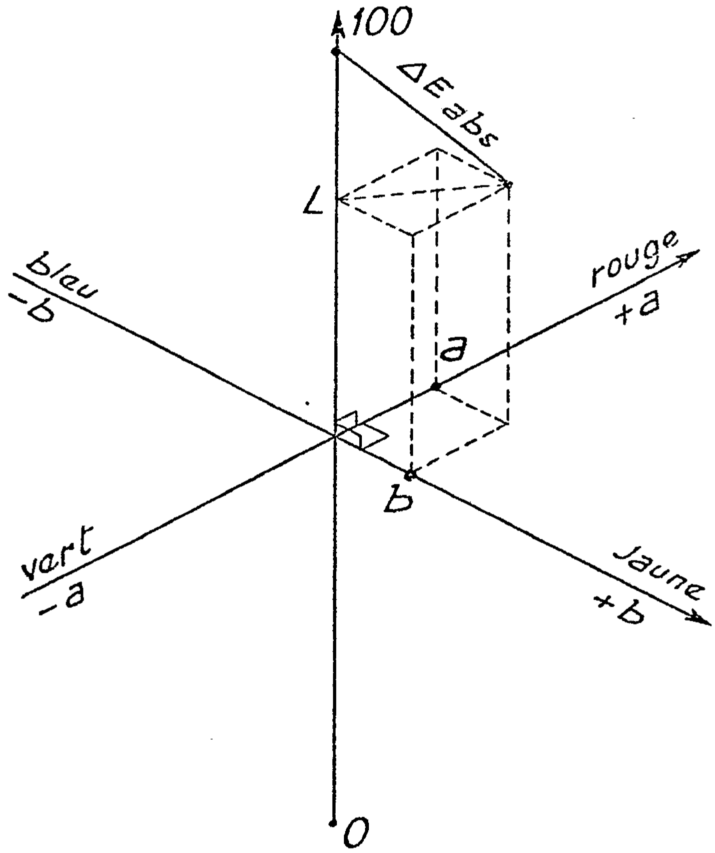
100
ΔE abs
L
bleu
-b
rouge
+a
a
b
vert
-a
Jaune
+b
0